# Europäisches Patentamt
# European Patent Office
# Office européen des brevets

(11) Publication number: **0 227 772**
**B1**

(12) **EUROPEAN PATENT SPECIFICATION**

(45) Date of publication of patent specification: **13.06.90**

(51) Int. Cl.⁵: **C 12 Q 1/68, C 07 H 21/00**

(21) Application number: **86904235.8**

(22) Date of filing: **17.06.86**

(86) International application number:
**PCT/GB86/00349**

(87) International publication number:
**WO 86/07612 31.12.86 Gazette 86/28**

(54) **NUCLEIC ACID SEQUENCING BY EXONUCLEASE INHIBITION.**

(30) Priority: **17.06.85 GB 8515276**

(43) Date of publication of application:
**08.07.87 Bulletin 87/28**

(45) Publication of the grant of the patent:
**13.06.90 Bulletin 90/24**

(84) Designated Contracting States:
**DE FR GB IT SE**

(56) References cited:
EP-A-0 123 513
US-A-4 521 509

Chemical Abstracts, volume 100, no. 7, 13 February 1984, (Columbus, Ohio, US), R.Wu et al: "Genetic Engineering technology - an overview and recent advances", see page 145, abstract no. 46293j, & NSC Symp. Ser. 1982, 4, 1-21

(73) Proprietor: **AMERSHAM INTERNATIONAL plc**
**Amersham Place**
**Little Chalfont Buckinghamshire HP7 9NA (GB)**

(72) Inventor: **MUNDY, Christopher**
**Amersham Place Little Chalfont**
**Bucks. HP7 9NA (GB)**
Inventor: **CUNNINGHAM, Martin**
**Amersham Place Little Chalfont**
**Bucks HP7 9NA (GB)**

(74) Representative: **Pennant, Pyers et al**
**Stevens, Hewlett & Perkins 5 Quality Court**
**Chancery Lane**
**London, WC2A 1HZ (GB)**

(56) References cited:

Chemical Abstracts, volume 105, no. 9, 1 September 1986, (Columbus, Ohio, US), S. Labeit et al.: "A new method of DNA sequencing using deoxynucleoside alpha - thiotriphosphates", see page 330, abstract no. 75347p, & DNA 1986 5(2), 173-7 (cited in the application)

Courier Press, Leamington Spa, England.

# EP 0 227 772 B1

(56) References cited:

Chemical Abstracts, volume 97, no. 1, 5 July
1982, (Columbus, Ohio, US), L. Guo et al.: "New
rapid methods for DNA sequencing based on
exonuclease III digestion followed by repair
synthesis", p. 162, abstract no. 1521k, & Nucl.
Acid. Res. 1982, 10(6), 2065-84

**Description**

Modern methods of RNA and DNA sequencing all depend on the same fundamental procedure: the resolution by polyacrylamide gel electrophoresis of nested sets of labelled single-stranded nucleic acid fragments with one defined end in common. Base-specific reactions, either chemical or enzymic, are used to ensure that the undefined ends occur only at bases of a particular and known type, and the reactions are controlled to ensure that in a population of molecules all appropriate termination points are represented. The distance from the defined end of each base of a given type is evident from the length of the nucleic acid fragment which it terminates. When all base-specific reactions are run side by side, the sequence from the defined end can be read by noting the base which terminates fragments of increasing size.

Three types of base-specific reaction are used in conventional nucleic acid sequencing methods:

1) The incorporation of chain-terminating nucleotides during nucleic acid synthesis by a suitable polymerase enzyme.

2) Nucleic acid cleavage by base-specific chemical reactions.

3) Nucleic acid cleavage by base-specific endonuclease enzymes.

Each of these approaches has characteristics which limit its usefulness in some sequencing applications.

Sanger and his co-workers pioneered the use of chain-terminating nucleotides for sequencing. Their method requires a single-stranded template, a primer, a suitable polymerase enzyme, and mixtures of normal and chain-terminating nucleotides, including a radioactive label.

The single-stranded template for DNA sequencing is usually generated as a clone of insert DNA in the single-stranded bacteriophage M13. The primer is usually a synthetic oligonucleotide which can hybridise to a region of the M13 vector adjacent to the insert to be sequenced. The polymerase enzyme adds nucleotides, one of which is labelled, to the 3' end of the primer, using the insert as a template, until a chain-terminating nucleotide is incorporated.

The primer defines a common 5' end for all labelled fragments in the mixture, and the nature of the terminating nucleotide defines the base at that position.

Single-stranded template may also be produced by heating linear double-stranded plasmid DNA, but reannealing of the two strands can interfere with the sequencing reactions. Double-stranded plasmids are used for most routine DNA manipulations and it is often necessary to check the results by sequencing. It is thus disadvantageous to clone the fragments of interest into M13. Sanger sequencing in M13 is also inherently undirectional, unless special measures are taken. The present invention permits the use of single or double-stranded templates, and permits sequencing of double-stranded templates in either direction with equal facility.

Maxam and Gilbert established the use of base-specific chemical cleavages for DNA sequencing, and defined the principles of sequencing by fractionation of nested fragments. Also, more recently, conditions have been defined which permit base-specific chemical cleavage of RNA. A number of chemical cleavages can be used, all of which involve a nucleic acid modification step and a chain scission step. Each modification step is specific for one base or class of base, and leads to increased lability of the modified base under certain conditions.

There are several facets of the Maxam and Gilbert method which make the Sanger method preferable for many applications. Chemicals which react with nucleic acids are usually very toxic and carcinogenic. Also, some of the chemicals in current use are quite unstable. They must be stored and used under closely controlled conditions. Furthermore, it is necessary that molecules to be sequenced chemically are labelled only at one end, to ensure that only fragments with one defined end in common are visualised. In the case of single-stranded DNA, it is not difficult to label either the 5' end or the 3' end, and so to visualise sets of nested fragments with either a 5' end or a 3' end in common. To obtain double-stranded DNA labelled at only one end of one strand, however, requires several manipulations and preferably the use of specialised vectors. The use of end-labelling rather than uniform labelling also reduces label density and increases the time required to obtain a result. The present invention avoids the use of dangerous and unstable chemicals, and permits convenient and uniform labelling of the nested fragments which are visualised to obtain sequence information.

Base specific endonuclease enzymes can be used only for RNA sequencing, because no suitable endodeoxyribonucleases have yet been discovered. Single-stranded RNA labelled at only one end is required.

Recently, there has been a major advance in DNA sequencing methodology. It has proved possible to transfer unlabelled gel-fractionated DNA sequencing reactions from the polyacrylamide gel to a membrane, and to visualise relevant DNA bands using labelled hybridisation probes. Church and Gilbert have applied the method to sequencing particular regions of mouse DNA directly, without isolating cloned fragments, following partial chemical cleavage of total mouse DNA, gel electrophoresis, transfer to a nylon membrane, and hybridisation with a labelled RNA probe. Similarly, Ward has subjected mixtures of up to 50 plasmid clones to Maxam and Gilbert chemical cleavages, and visualised sequence patterns for individual clones with clone-specific labelled RNA hybridisation probes. Labelled probe can be removed following visualisation. Successive probes can be generated very conveniently and applied to reveal sequencing patterns for a large number of clones following a single set of sequencing reactions. Thus the

3

amount of information which can be gleaned from a single gel can be increased greatly by using labelled hybridisation probes to visualise sequencing reactions. Furthermore, this approach permits the use of a great variety of labelling methods, including non-radioactive methods, because the limitations which apply to the labelling of sequencing reactions do not apply to the labelling of hybridisation probes. Fully automated large-scale sequencing may be developed using this approach.

There is one property of the hybridisation-based sequencing method which limits the suitability of the conventional methods of nested fragment generation. Labelled hybridisation probes for this application are generated by transcribing a short length of the insert into RNA, from a promoter on the plasmid vector. RNA (and DNA) is of necessity synthesised in the 5' to 3' direction. For hybrid-visualised sequencing to work, complementary sequences on the filter must share a common defined 3' end, rather than a common defined 5' end. The Sanger method cannot be used in this application without a number of complex and impractical manipulations. The Maxam and Gilbert method can and has been used, because conditions can be applied which give rise to nested fragments with a common 3' end, complementary to the hybridisation probe. The aforesaid disadvantages of the chemical sequencing method still apply, however, in this application.

The present invention permits production of sets of nested fragments with either defined 3' or defined 5' ends in common, with equal facility, in rapid one-tube reactions. Thus, as well as providing the aforesaid advantages of facility of use with double-stranded templates, enzymic reactions and uniform labelling, the present invention can be applied readily to hybridisation-based sequencing.

References

Sanger F., Nicklen S. and Coulson A. R., Proc. Natl. Acad. Sci. U.S.A., Vol 74, 1977, page 5463—7.
Maxam A. M. and Gilbert W., Proc. Natl. Acad. Sci. U.S.A., Vol 74, 1977, pages 560—4.
Church G. M. and Gilbert W., Proc. Natl. Acad. Sci. U.S.A., Vol 81, 1984, pages 1991—5.

The Invention

The present invention is based on the concept of introducing into a nucleic acid chain to be sequenced a thio-nucleotide that is resistant to digestion under particular conditions, and then digesting the chain under those particular conditions to obtain a nested set of fragments with one defined end in common.

Thus the invention provides a method of sequencing polynucleotides by:

i) providing a nucleic acid chain having a double-stranded section to be sequenced of which one strand is the target and the other is a synthetic strand including at one or more positions a thio-nucleotide that is resistant to digestion under particular conditions,

ii) digesting the synthetic strand by means of an exonuclease under the said particular conditions whereby digestion is arrested at positions occupied by the said nucleotide derivative,

iii) if not already present, providing a defined end common to all fragments of the synthetic strand, step iii) being performed either before or after step ii),

iv) size-fractioning the resulting mixture of fragments.

Crucial to the invention is the thio-nucleotide used in step i). The thio-nucleotide may in principle be a nucleotide which has been modified in the sugar, or in the base, or in the phosphate group that becomes involved in the phosphodiester bond. Many such modified nucleotides have been described in the literature. A suitable thio-nucleotide in many instances is one in which an oxygen atom attached to the alpha-phosphorus atom has been replaced by sulphur, for example.

alpha-S-deoxythymidine triphosphate (alpha-SdTTP)
alpha-S-deoxyadenosine triphosphate (alpha-SdATP)
alpha-S-deoxycytidine triphosphate (alpha-SdCTP)
alpha-S-deoxyguanosine triphosphate (alpha-SdGTP)

The thio-nucleotide needs to be chosen in conjunction with the particular digestion conditions (which in practice often means the particular exonuclease) used in step ii). One suitable enzyme is exonuclease III from E. coli which digests double-stranded DNA only from the 3' end, releasing deoxy-nucleoside-5'-monophosphates. This enzyme cannot cleave phospho-ester bonds when the phosphorus atom is linked to sulphur. Another enzyme, suitable in different circumstances, is Lambda exonuclease from phage Lambda-infected E. coli, which digests double-stranded DNA only from the 5' end, releasing deoxy-nucleoside-5'-monophosphates. It cannot cleave phosphodiester bonds when the phosphorus atom is linked to sulphur. Exonuclease III is double-strand specific and thus will not degrade double-stranded DNA from an overhanging 3' end. Lambda exonuclease, however, will degrade single-stranded DNA, albeit at a lower rate than double-stranded DNA. Thus it will degrade double-stranded DNA from blunt or overhanging ends. Another enzyme which digests double-stranded DNA from the 5' end and may be useful in different circumstances is phage T7 gene 6 exonuclease.

Size-fractionation of the mixture of fragments in step iv) may be effected for example by denaturing polyacrylamide gel electrophoresis. Visualisation of fragments sharing a common defined end and terminated by a thio-nucleotide as described, may be effected by means of a radioactive or other label incorporated during synthesis of the synthetic strand. Alternatively, visualisation may be effected by hybridisation with a labelled probe, for example as described by Church and Gilbert.

The invention is mainly, though not exclusively, applicable to the sequencing of single-stranded or double-stranded DNA. To sequence RNA it would be necessary to (i) synthesise a complementary DNA strand with reverse transcriptase and (ii) modify the RNA with base-specific reactions which would permit arrest of exonuclease digestion.

The invention will be further described with reference to eight preferred embodiments denoted A to H. In this connection, reference is directed to the accompanying drawings in which:

| Figure | 1 relates to Embodiment | A, |
|---|---|---|
| | 2 | B, |
| | 3 | C, |
| | 4 | D, |
| | 5 | E, |
| | 6 | F, |
| | 7 | G, and |
| | 8 | H. |

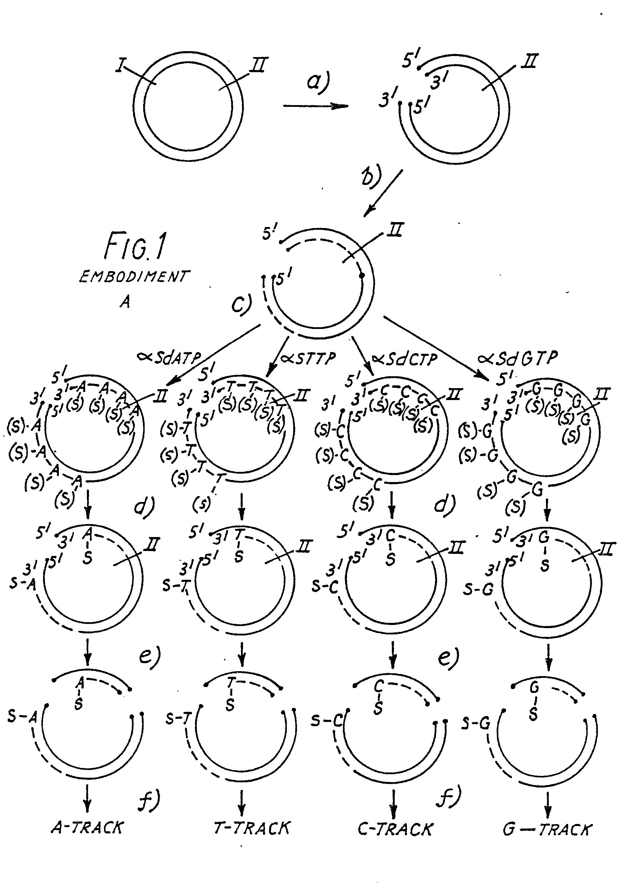

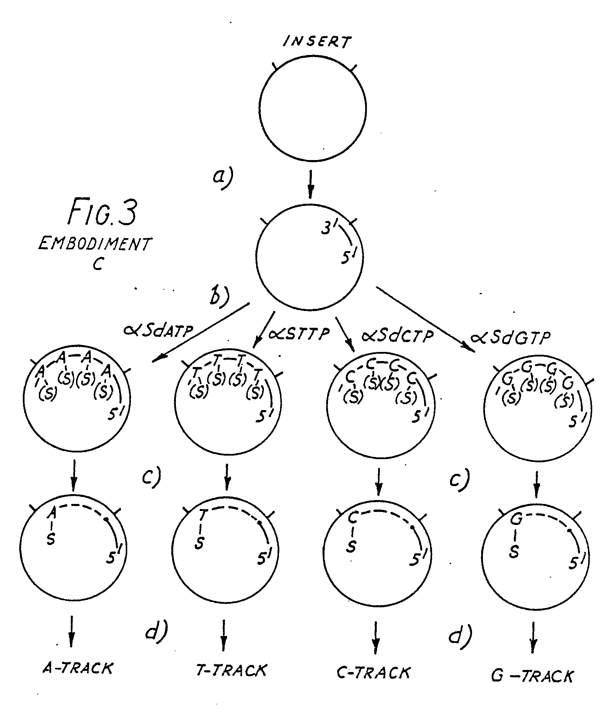

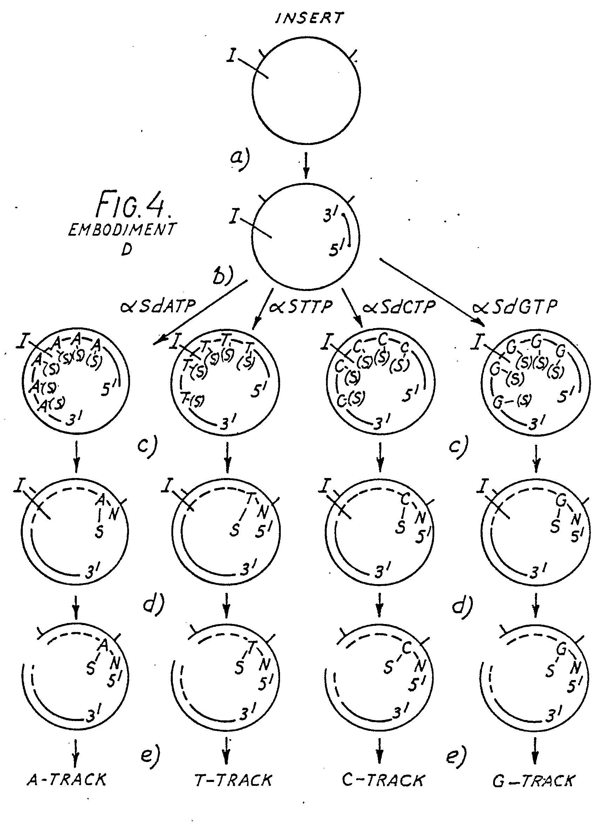

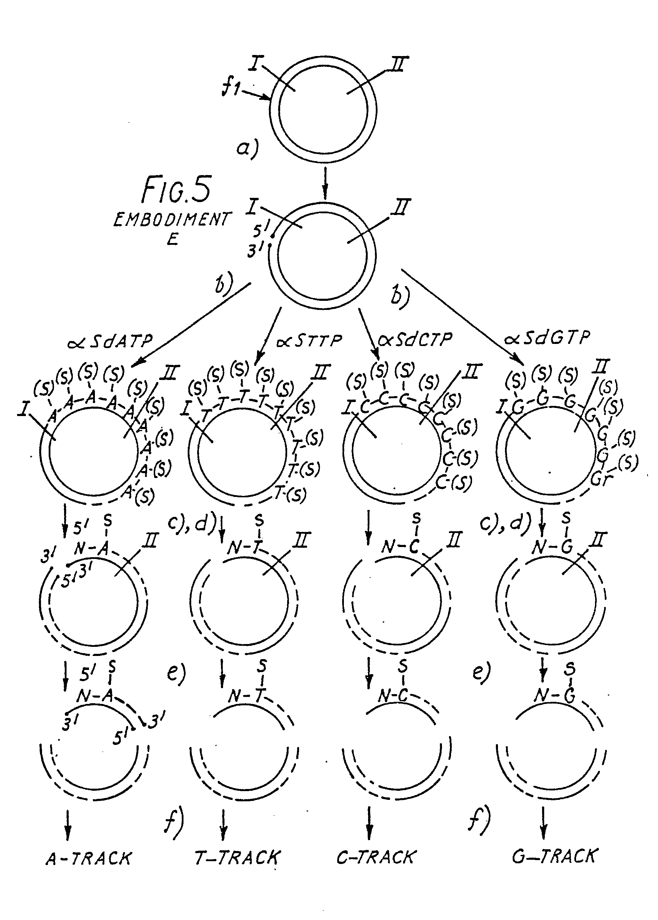

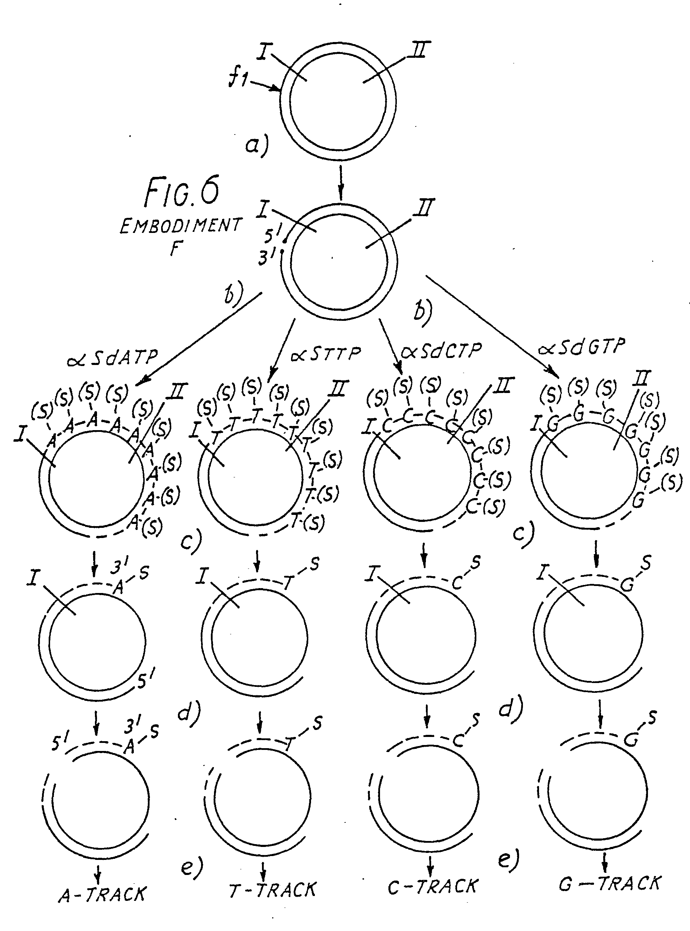

In Figures 1 to 6, the DNA is shown as being circular, and single-stranded or double-stranded as appropriate, on which the section to be sequenced extends from about 10 o'clock to about 2 o'clock. Restriction sites are denoted by the roman numerals I, II and III. Dashed lines indicate undefined extend of DNA strands, as a result of extension or degradation.

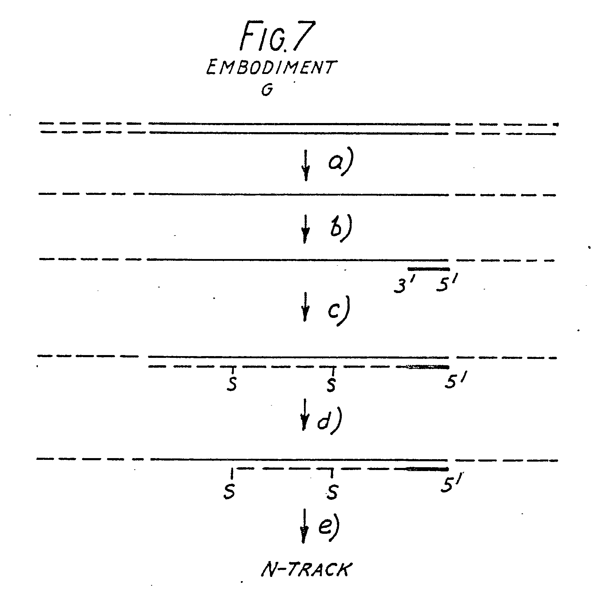

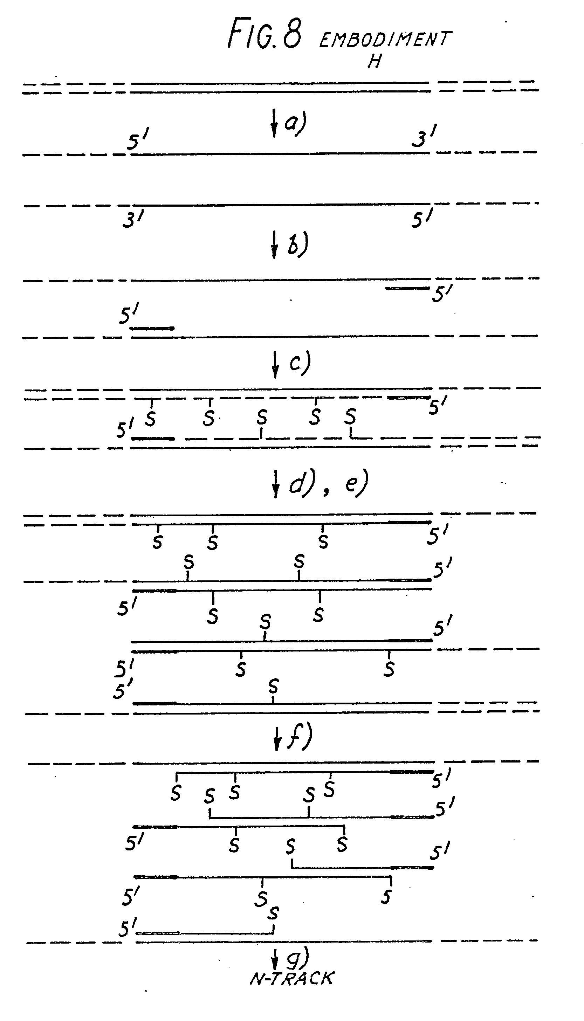

In Figures 7 and 8 the DNA is shown as being linear, and the primer is shown as a heavy line to distinguish it from the synthetic strand extending in the 3' direction from it.

The symbol (S) indicates a unit that may be a nucleotide or a thio-nucleotide. In Embodiments E and F, the nick is indicated by a gap immediately following the nicking reaction, and by a dash after the extension reaction to indicate that its position is undefined. In each figure, the letters a), b) etc. relate to the corresponding steps as described below.

A. This embodiment starts with double-stranded DNA including a section to be sequenced and comprises the following steps

i) a) cleaving the DNA at a position adjacent one end of the section to be sequenced to make the 3' end of one strand accessible to an exonuclease, e.g. exonuclease III, which is specific for 3' ends of double-stranded DNA,

i) b) digesting the cleaved DNA with the specific exonuclease for a sufficient time to render the section to be sequenced single-stranded,

i) c) adding nucleotides, including a thionucleotide which is resistant to digestion under particular conditions, to the reaction mixture and effecting polymerisation so as to re-synthesise the DNA removed in step b) and generate a synthetic strand including the thio-nucleotide at one or more positions,

ii) d) digesting the synthetic strand with an exonuclease that attacks the 3' end of the chain under the particular conditions such that digestion is arrested at positions occupied by the thio-nucleotide,

iii) e) cleaving the DNA to produce a defined 5' end common to all fragments of the synthetic strand, and

iv) f) size fractionating the mixture of fragments resulting from step e).

In step a), the restriction enzyme should not leave a 3' overhang, because a single-stranded end is resistant to exonuclease III digestion in step b).

In step c), the reaction mixture containing the target is preferably divided into four parts and the nucleotides added to each, a different thio-nucleotide being added to each part. All four nucleotides are added to each part together with a thio-nucleotide. The proportion of thio-nucleotide (e.g. alpha — SdATP) to the related nucleotide (e.g. dATP) is chosen to ensure that some, but not all, A positions in the synthetic strand are occupied by the thio-nucleotide. Also in step c), one of the nucleotides added is preferably labelled e.g. with 32-P, to provide a means for visualising the fractionated fragments resulting from step f). Alternatively, a 35-S-labelled thio-nucleotide may be used.

In step d) the synthetic DNA strands are digested e.g. with exonuclease III. After digestion, all the synthetic strands containing one of the four types of thio-nucleotide terminate at their 3' ends with such a thio-nucleotide.

In step e), a common defined 5' end is generated, preferably by digestion with a restriction enzyme chosen to cleave the DNA at a position adjacent the other end [in relation to the end referred to in step a)] of the section to be sequenced.

B. This embodiment starts with double-stranded DNA including a section to be sequenced. Unlike embodiment A, it generates a nested set of fragments with a common defined 3' end. This has the

5

advantage that it facilitates subsequent hybridisation of the fragments with a labelled probe in the manner described by Ward. The method comprises the following steps:—

i) a) cleaving the DNA at a position adjacent one end of the section to be sequenced to make the 3' end of one strand accessible to an exonuclease, e.g. exonuclease III, which is specific for 3' ends of double-stranded DNA,

i) b) digesting the cleaved DNA with the specific exonuclease for a sufficient time to render the section to be sequenced single-stranded,

i) c) adding nucleotides, including a thio-nucleotide which is resistant to digestion under particular conditions, to the reaction mixture and effecting polymerisation so as to re-synthesise the DNA removed in step b) and generate a synthetic strand including the thio-nucleotide at one or more positions,

iii) d) cleaving the DNA, e.g. by digestion with restriction enzymes, at positions adjacent both ends of the section to be sequenced,

ii) e) digesting the synthetic strand with an exonuclease, e.g. lambda exonuclease, that attacks the 5' end of the chain under the particular conditions such that digestion is arrested at positions occupied by the thio-nucleotide,

iv) f) size fractionating the mixture of fragments resulting from step e).

Again, the restriction enzyme used in step a) should not leave a 3' overhang.

In step c), the reaction mixture is preferably divided into four parts as described in embodiment A. One of the nucleotides used in that step may be labelled; alternatively, the fragments from step f) may be visualised by means of a labelled probe. The exonuclease is preferably Lambda exonuclease. In this case, all synthetic strands containing one of the four types of thio-nucleotide terminate at their 5' ends precisely one nucleotide away from a thio-nucleotide. If re-synthesis in step c) was complete, or optionally following a further restriction enzyme digestion following step e), all fragments of the synthetic strand have a common defined 3' end. The positions of the thio-nucleotides are indicated by the lengths of the digested fragments.

C. In this embodiment, the template to be sequenced is single-stranded DNA, for example an insert cloned in M13. The method comprises the following steps:

i) a) annealing an oligonucleotide primer to the vector adjacent the 3' end of the insert,

i) b) adding nucleotides, including a thionucleotide which is resistant to digestion under particular conditions, to the reaction mixture and effecting polymerisation so as to generate a synthetic strand complementary to the insert including the thio-nucleotide at one or more positions,

ii) c) digesting the synthetic strand with an exonuclease that attacks the 3' end of the chain under the particular conditions such that digestion is arrested at positions occupied by the thio-nucleotide,

iv) d) size fractionating the mixture of fragments resulting from step c).

Because the fragments generated in step c) share a common defined 5' end, no cleavage is needed between steps c) and d).

S. Labeit et al. (DNA, Vol. 5, No. 2, 1986 pages 173—7) provides further information in relation to this embodiment.

D. This embodiment starts with single-stranded DNA, for example an insert cloned in M13. Unlike embodiment C it generates a nested set of fragments with a common defined 3' end. The method comprises the following steps:

i) a) annealing an oligonucleotide primer to the vector adjacent the 3' end of the insert,

i) b) adding nucleotides, including a thio-nucleotide which is resistant to digestion under particular conditions, to the reaction mixture and effecting polymerisation so as to generate a synthetic strand complementary to the insert including the thio-nucleotide at one or more positions,

ii) c) digesting the synthetic strand with an exonuclease that attacks the 5' end of the chain under the particular conditions such that digestion is arrested at positions occupied by the thio-nucleotide,

iii) d) cleaving the DNA, e.g. by digestion with a restriction enzyme, at a position adjacent the 5' end of the insert, to produce a defined 3' end common to all fragments of the synthetic strand,

iv) e) size fractionating the mixture of fragments resulting from step d).

The chain extension in step b) should be long enough to ensure that labelled fragments extending beyond the insert do not interfere with the sequence pattern.

E. This embodiment starts with double-stranded DNA and comprises the following steps:—

i) a) forming a nick in one strand, e.g. by means of phage f1 protein A at a site, defined by the recognition sequence of that protein, adjacent one end of the section to be sequenced,

i) b) adding nucleotides, including a thio-nucleotide which is resistant to digestion under particular conditions, to the reaction mixture and effecting nick translation so as to generate a synthetic strand extending the length of the section to be sequenced and including the thio-nucleotide at one or more positions,

i) c) cleaving the DNA, e.g. by digestion with a restriction enzyme, at a site adjacent that at which the nick was formed in step a),

ii) d) digesting the synthetic strand with an enzyme that attacks the 5' end, e.g. lambda exonuclease, the digestion being performed under the particular conditions such that it is arrested at positions occupied by the thio-nucleotide,

iii) e) cleaving the DNA at a site adjacent the other end (with respect to that referred to in step a) ) of the section to be sequenced, and

iv) f) size fractionating the mixture of fragments having a common defined 3' end resulting from step e).

Step b) should preferably be continued until most nicks are well beyond the section to be sequenced. Alternatively, step e) could be performed prior to step a).

F. This embodiment starts with double stranded DNA. Unlike embodiment E, there is generated a mixture of fragments having a common defined 5' end. The embodiment comprises the following steps:—

i) a) forming a nick in one strand, e.g. by means of phage f1 protein A at a site, defined by the recognition sequence of that protein, adjacent one end of the section to be sequenced,

i) b) adding nucleotides, including a thio-nucleotide which is resistant to digestion under particular conditions, to the reaction mixture and effecting nick translation so as to generate a synthetic strand extending the length of the section to be sequenced and including the thio-nucleotide at one or more positions,

i) c) digesting the synthetic strand with an enzyme that attacks the 3' end at the nick after translation, e.g. exonuclease III, the digestion being performed under the particular conditions such that it is arrested at positions occupied by the thio-nucleotide,

iii) d) cleaving the DNA at a site adjacent that at which the nick was formed in step a), and

iv) e) size fractionating the mixture of fragments having a common defined 5' end resulting from step d).

G. This embodiment starts with double-stranded DNA including a target region. The procedure resembles that of C above and comprises the following steps:—

i) a) denaturing the double-stranded starting DNA,

i) b) annealing an oligonucleotide primer adjacent the 3' end of the target region of one strand,

i) c) adding nucleotides, including a thio-nucleotide which is resistant to digestion under particular conditions, to the reaction mixture and effecting polymerisation so as to generate a synthetic strand complementary to the target region including the thio-nucleotide at one or more positions,

ii) d) digesting the synthetic strand with an exonuclease that attacks the 3' end of the chain ' under the particular conditions such that digestion is arrested at positions occupied by the thio-nucleotide,

iv) e) size fractionating the mixture of fragments resulting from step d).

This method is in principle suitable for sequencing genomic DNA rather than DNA which has been purified by cloning. It would hardly be feasable to incorporate radiolabel during this reaction, because such label would be incorporated at many sites due to annealing of fold-back and repeated sequences. The nested sequences could, however, be visualised by electrophoresis, blotting to a solid support, and hybridisation with a highly labelled probe complementary to the primer. Unlike the Ward blot sequencing application mentioned above, it is not necessary to sequence from and generate probe from the same clone. There is no conflict between the orientation of the primer and the probe, and a 5'—3' exonuclease is not required. It is possible to generate a high specific activity RNA probe complementary to the primer simply by using a suitable clone in a vector containing an SP6 or T7 promoter.

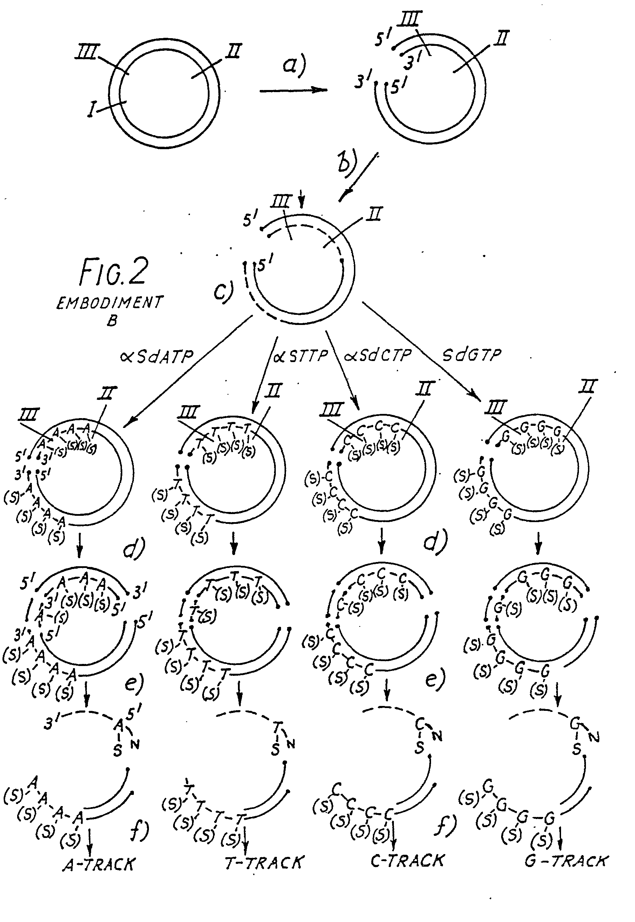

H. R. K. Saiki et al have described (Science, 230 (4732), Dec. 1985 page 1350—4) a method for amplifying specified regions of genomic DNA. Two primers which anneal to opposite strands at either end of the target sequence are used to prime DNA synthesis on denatured genomic DNA, in the presence of dNTPs and "Klenow" polymerase. Following this reaction the mixture is heated to denature the new DNA, the same primers are allowed to anneal to both the original two strands and the new strands, more enzyme is added, and the amount of target sequence doubles again (see Figure 2). This denaturation-synthesis cycle is repeated as often as described, and can lead to amplifications of more than $10^5$. It should be noted that after the first extension reaction, only those molecules which are more or less completely resynthesised will be amplified further, because the priming site for the next round of synthesis must be copied.

This embodiment is a variation of G. above which makes use of this amplification procedure. The embodiment comprises the following steps:—

i) a) denaturing double-stranded starting DNA,

i) b) annealing oligonucleotides primers adjacent the 3' ends of the target regions of both strands,

i) c) adding nucleotides, including a thio-nucleotide which is resistant to digestion under particular conditions, to the reaction mixture and effecting polymerisation so as to generate synthetic strands complementary to the target regions of both strands including the thio-nucleotide at one or more positions,

i) d) denaturing the resulting double-stranded DNA,

i) e) repeating steps i) b) and i) c) and i) d) in sequence as often as desired to achieve a desired concentration of target region,

ii) digesting the synthetic strands with an exonuclease that attacks the 3' end of the chain under the particular conditions such that digestion is arrested at positions occupied by the thionucleotide,

iv) g) size fractionating the mixture of fragments resulting from step f).

Inclusion of a suitable level of a particular SdNTP throughout such an amplification reaction, followed by exonuclease III digestion, leads to a highly amplified population of molecules with 5' ends defined by the two primers used in the amplification reaction, and 3' ends defined by the particular SdNTP. The large amounts of target DNA permit visualisation of nested fragments with end-labelled oligonucleotide hybridisation probe complementary to one or other of the primers, after electrophoresis and blotting. Alternatively, it may be possible to use one or other of the primers labelled, perhaps non-radioactively, throughout the amplification reactions, and avoid the blotting and hybridisation steps.

In summary, the thio-sequencing method should be applicable to genomic sequencing without amplification (G), but its applicability should be enhanced greatly by amplification (H). In particular amplification may permit non-radioactive genomic sequencing.

In many cases, procedures can be simplified by conducting reactions simultaneously.

These and many other variations of the procedures described in the eight embodiments are possible according to the invention and will readily occur to the skilled reader.

## Example 1

0.5 µg pAT153 was mixed with 4 units of Bam H 1 and 10 units of exonuclease III in

30 µl of 125 mM NaCl
50 mM Tris HCl pH 7.5
10 mM $MgCl_2$
5 mM dithiothreitol

and incubated at room temperature for 45 minutes. The tube was heated at 70°C for 10 minutes. 15 µl was transferred to another tube, and nucleotides were added to the following final concentrations in a final volume of 18 µl:

200 µM dGTP
200 µM dTTP
200 µM dATP
50 µM α SdATP

This volume also contained 10 microcuries of $\alpha^{32}P$ dCTP, specific activity ca. 3000 curies per millimole, and 4 units of E. coli DNA polymerase I "Klenow" fragment.

The mixture was incubated for 10 minutes at room temperature. 1µl of 5 mM dCTP was added, and the mixture was incubated for a further 15 minutes at room temperature.

The tube was heated at 70°C for 10 minutes and 10 units of exonuclease III were added in a volume of 1 µl. The mixture was incubated at room temperature for 1 hour. Following another heat inactivation step (70°C, 10 minutes), 1 µl of Cla 1 solution containing 5 units was added and the mixture was incubated at 37°C for 15 minutes.

20 µl of the following solution was added:

3 mg bromophenol blue
3 mg xylene cyanol
20 µl 0.5M EDTA pH 8
1 ml deionised formamide

A 1 µl aliquot was subjected to electrophoresis on a 5% polyacrylamide gel containing 7M urea, and an autoradiograph was taken on Fuji X-ray film. The resulting autoradiograph represented an "A-track" extending from near to the Cla 1 site at the bottom of the gel towards the Bam H 1 site of pAT153, which was an accurate representation of the known sequence of the section.

## Example 2

Example Sequencing of double-stranded DNA using SdNTPs and exonuclease III.

$^{32}p$ Label

A partial sequence of supercoiled plasmid pBR322, the region between BamHI and Hind III sites, was obtained in the following way.

1. 3 µg of pBR322 were mixed with 6 units of restriction endonuclease BamHI and 20 units of E. coli exonuclease III in the presence of 50 mM Tris HCl pH 7.5, 75 mM NaCl, 10 mM $MgCl_2$ and 5 mM dithiothreitol, in a final volume of 20 µl. The mixture was incubated at 37°C for 45 minutes.

2. The reaction was stopped and the enzymes inactivated by heating at 70°C for 10 minutes.

3. 10 µl of the mixture from step 2 was mixed with 6 µl of 100 mM Tris HCl pH 8.0, 50 mM Mg Cl₂, 4 units of E. coli DNA polymerase, "Klenow" fragment, and made to 60 µl with water.

4. The mixture from step 3 was divided into four 15 µl volumes designated A, G, C and T. 15 microcuries of α32 P — dCTP, 3000 curies per milimole, was added to each reaction, followed by 2 µl of the appropriate nucleotide mixture listed below:

| A | G | C | T |
|---|---|---|---|
| 1 mM dGTP | 1 mM dATP | 1 mM dATP | 1 mM dATP |
| 1 mM TTP | 1 mM TTP | 1 mM dGTP | 1 mM dGTP |
| 50 µM dCTP | 50 µM dCTP | 1 mM TTP | 50 µM dCTP |
| 850 µM dATP | 850 µM dGTP | 42.5 mM dCTP | 950 µM TTP |
| 150 µM dATPαS | 150 µM dGTPαS | 7.5 mM dCTPαS | 50 µM TTPαS |

The mixtures were incubated at room temperature for 10 minutes.

5. 1 µl 2 mM dCTP was added to the A, G and T reactions, and 1 µl of 1.4 mM dCTP, 0.6 mM dCTPαS was added to the C reaction. The mixtures were incubated at room temperature for a further 10 minutes.

6. The reaction was stopped and the enzyme inactivated by incubation at 70°C for 10 minutes.

7. 1.5 µl of 750 mM NaCl, 500 mM Tris HCl pH 7.5, 100 mM $MgCl_2$, 50 mM dithiothreitol, and 2 units of exonuclease III were added to each reaction. The mixtures were incubated at 37° for 30 minutes.

8. The reactions from step 7 were incubated at 70° for 10 minutes to inactivate the enzyme.

9. 5 units of restriction endonuclease Hind III were added to each reaction from step 8, and the mixtures were incubated at 37° for 15 minutes.

10. Formamide — containing tracker dye was then added to each tube, and aliquots were subjected to electrophoresis on a 6% polyacrylamide gel under denaturing conditions.

11. Autoradiography was used to reveal the labelled DNA fragments in the gel. It was possible to read an accurate sequence of most of the region of plasmid pBR322 between the Bam HI and Hind III sites.

Example 3
Sequencing of double-stranded DNA using SdNTPs and exonuclease III
$^{35}$S label

The protocol for this experiment was as for Example 2, except that the following step replaced steps 4 and 5 of that Example.

4,5. The mixture from step 3 was divided into four 15 µl volumes, designated A, G, C and T. 15 microcuries of [α-$^{35}$S] dATP S, specific activity 1000 curies per millimole, was added to the A reaction, and the same quantity of [α-$^{35}$S] dGTP S, [α-$^{35}$S]-dCTP S and [α-$^{35}$S] TTp S to the G, C and T-reactions, respectively. Also, 2 µl of the following mixtures were added, the A mixture to the A-reaction etc.

| A | G | C | T |
|---|---|---|---|
| 1 mM dGTP | 1 mM dATP | 1 mM dATP | 1 mM dATP |
| 1 mM TTP | 1 mM TTP | 1 mM TTP | 1 mM dGTP |
| 1 mM dCTP | 1 mM dCTP | 1 mM dGTP | 1 mM dCTP |
| 40 µM dATP | 80 µM dGTP | 20 µM dCTP | 200 µM TTP |

The reactions were incubated at room temperature for 10 minutes.

Following autoradiography as in step 11 of Example 2, it was again possible to read an accurate sequence of most of the region of plasmid pBR322 between the BamHI and Hind III sites.

**Claims**

1. A method of sequencing polynucleotides by:
i) providing a nucleic acid chain having a double-stranded section to be sequenced of which one strand is the target and the other is a synthetic strand including at one or more positions a thio-nucleotide that is resistant to digestion under particular conditions,
ii) digesting the synthetic strand by means of an exonuclease under the said particular conditions whereby digestion is arrested at positions occupied by the said thio-nucleotide,
iii) if not already present, providing a defined end common to all fragments of the synthetic strand, step iii) being performed either before or after step ii),
iv) size-fractioning the resulting mixture of fragments.

2. A method as claimed in claim 1, wherein the exonuclease is exonuclease III or Lambda exonuclease.

3. A method as claimed in claim 1 or claim 2, wherein the synthetic strand is formed by the use of nucleotides, including a thio-nucleotide, under polymerisation conditions.

4. A method as claimed in claim 3, wherein a reaction mixture containing the target is divided into four parts and the nucleotides added to each, a different thio-nucleotide being added to each part.

5. A method as claimed in any one of claims 1 to 4, wherein the polynucleotide to be sequenced is double-stranded DNA, which is digested with an exonuclease enzyme to provide the target.

6. A method as claimed in any one of claims 1 to 4, wherein the polynucleotide to be sequenced is single-stranded DNA, and an oligonucleotide primer is annealed adjacent the 3' end thereof.

7. A method as claimed in any one of claims 1 to 4, wherein the polynucleotide to be sequenced is double-stranded DNA, and the synthetic strand is introduced by nick translation in the presence of nucleotides including the thio-nucleotide.

8. A method as claimed in any one of claims 1 to 4, wherein the polynucleotide to be sequenced is double-stranded DNA including a target region, and the double stranded DNA is denatured and an oligonucleotide primer is annealed adjacent the 3' end of one or each strand.

9. A method as claimed in claim 8, wherein oligonucleotide primers are annealed adjacent the 3' end of both strands, synthetic strands are formed by the use of nucleotides, including a thio-nucleotide, under polymerisation conditions, the resulting double-stranded DNA is denatured, and the steps of annealing, polymerisation and denaturing are repeated to amplify the target region.

10. A method as claimed in claim 8 or claim 9, wherein the polynucleotide to be sequenced is genomic DNA.

11. A method as claimed in any one of claims 1 to 10, wherein size fractionation of the mixture of fragments is effected in step iv) by denaturing gel electrophoresis.

12. A method as claimed in claim 11, wherein visualisation of the fragments is effected by means of a label incorporated during synthesis of the synthetic strand.

13. A method as claimed in claim 11, wherein visualisation of the fragments is effected by hybridisation with a labelled probe.

## Patentansprüche

1. Verfahren zum Sequenzieren von Polynukleotiden durch:

i) Bereitstellen einer Nukleinsäurekette mit einem doppelsträngigen zu sequenzierenden Bereich, dessen einer Strang das Ziel ist und der andere ein synthetischer Strang ist, der an einer oder mehreren Positionen ein Thio-Nukleotid aufweist, das gegenüber Verdauung unter besonderen Bedingungen beständig ist,

ii) Verdauen des synthetischen Stranges mit Hilfe einer Exonuklease unter den besonderen Bedingungen, wobei die Verdauung an Positionen, die durch diese Thio-Nukleotide besetzt sind, zum Stillstand gebracht wird,

iii) Wenn nicht bereits vorhanden, zur Verfügung stellen eines definierten Endes, das allen Fragmenten des synthetischen Stranges gemeinsam ist, wobei Schritt iii) entweder vor oder nach Schritt ii) durchgeführt wird,

iv) Auftrennen entsprechend der Größe der erhaltenen Mischung der Fragmente.

2. Verfahren nach Anspruch 1, worin die Exonuklease Exonuklease III oder Lambda-Exonuklease ist.

3. Verfahren nach Anspruch 1 oder 2, worin der synthetische Strang unter Verwendung von Nukleotiden, einschließlich eines Thio-Nukleotids, unter Polymerisationsbedingungen gebildet wird.

4. Verfahren nach Anspruch 3, worin eine Reaktionsmischung enthaltend das Ziel in vier Teile geteilt wird und die Nukleotide zu jedem zugegeben werden, wobei ein unterschiedliches Thio-Nukleotid zu jedem Teil zugegeben wird.

5. Verfahren nach einem der Ansprüche 1—4, worin das zu sequenzierende Polynukleotid doppelsträngige DNS ist, die mit einem Exonukleaseenzym verdaut wird, um das Ziel bereitzustellen.

6. Verfahren nach einem der Ansprüche 1—4, worin das zu sequenzierende Polynukleotid einzelsträngige DNS ist, und ein Oligonukleotid-Anfangsstück benachbart zu dem 3'-Ende davon gebunden wird.

7. Verfahren nach einem der Ansprüche 1—4, worin das zu sequenzierende Polynukleotid doppelsträngige DNS ist und der synthetische Strang durch nick translation in Gegenwart von Nukleotiden, einschließlich des Thio-Nukleotids eingeführt wird.

8. Verfahren nach einem der Ansprüche 1—4, worin das zu sequenzierende Polynukleotid doppelsträngige DNS einschließlich einer Zielregion ist und die doppelsträngige DNS denaturiert wird und ein Oligonukleotid-Anfangsstück neben dem 3'-Ende von einem oder jedem Strang gebunden wird.

9. Verfahren nach Anspruch 8, worin Oligonukleotid-Anfangsstücke benachbart zu den 3'-Enden von beiden Strängen gebunden werden, synthetische Stränge durch die Verwendung von Nukleotiden einschließlich eines Thio-Nukleotids unter Polymerisationsbedingungen gebildet werden, die entstehende doppelsträngige DNS denaturiert wird und die Schritte des Bindens, der Polymerisation und Denaturierung wiederholt werden um die Zielregion zu amplifizieren.

10. Verfahren nach Anspruch 8 oder 9, worin das zu sequenzierende Polynukleotid genomische DNS ist.

11. Verfahren nach einem der Ansprüche 1—10, worin die Auftrennung der Größe nach einer Mischung von Fragmenten in Schritt iv) durch denaturierende Gelektrophorese bewirkt wird.

12. Verfahren nach Anspruch 11, worin die Sichtbarmachung der Fragmente mit Hilfe einer Markierung bewirkt wird, die während der Synthese des synthetischen Stranges eingearbeitet wird.

13. Verfahren nach Anspruch 11, worin die Sichtbarmachung der Fragmente durch Hybridisierung mit einer markierten Sonde bewirkt wird.

**Revendications**

1. Une méthode de séquençage de polynucléotides par:
i) apport d'une chaîne d'acide nucléique ayant une partie bicaténaire à séquencer, dont un brin est la cible et l'autre un brin synthétique comprenant à une ou plusieurs positions un thio-nucléotide qui résiste à la digestion dans des conditions particulières,
ii) digestion du brin synthétique avec une exonucléase dans ces conditions particulières, digestion qui est arrêtée aux positions occupées par le thio-nucléotide,
iii) si elle n'est pas déjà présente, apport d'une extrémité définie commune à tous les fragments du brin synthétique, cette étape étant exécutée avant ou après l'étape ii), et
iv) fractionnement par taille du mélange de fragments résultant.

2. Une méthode selon la revendication 1 dans laquelle l'exonucléase est l'exonucléase III ou l'exonucléase lambda.

3. Une méthode selon la revendication 1 ou 2 dans laquelle le brin synthétique est formé avec des nucléotides, comprenant un thio-nucléotide, dans des conditions de polymérisation.

4. Une méthode selon la revendication 3 dans laquelle on divise un mélange de réaction contenant la cible en quatre parties auxquelles on ajoute les nucléotides, un thio-nucléotide différent étant ajouté à chaque partie.

5. Une méthode selon l'une quelconque des revendications 1 à 4, dans laquelle la polynucléotide à séquencer est de l'ADN à double brin (bicaténaire), que l'on fait digérer avec une exonucléase pour obtenir la cible.

6. Une méthode selon l'une quelconque des revendications 1 à 4', dans laquelle le polynucléotide à séquencer est de l'ADN monocaténaire auquel on apparie une amorce d'oligonucléotide à côté de son extrémité 3'.

7. Une méthode selon l'une quelconque des revendications 1 à 4, dans laquelle le polynucléotide à séquencer est de l'ADN bicaténaire et le brin synthétique est introduit par translation de l'encoche en présence de nucléotides comprenant le thio-nucléotide.

8. Une méthode selon l'une quelconque des revendications 1 à 4, dans laquelle le polynucléotide à séquencer est de l'ADN bicaténaire comprenant une région cible, l'ADN bicaténaire est dénaturé et on apparie une amorce d'oligonucléotide en position adjacente à l'extrémité 3' d'un brin ou de chaque brin.

9. Une méthode selon la revendication 8, dans laquelle les amorces d'oligonucléotides sont appariées en position adjacente à l'extrémité 3' des deux brins, on forme des brins synthétiques avec des nucléotides, comprenant un thio-nucléotide, dans des conditions de polymérisation, l'ADN bicaténaire formé est dénaturé, et on répète les étapes d'appariement, de polymérisation et de dénaturation pour amplifier la région cible.

10. Une méthode selon la revendication 8 ou 9, dans laquelle le polynucléotide à séquencer est un ADN de génome.

11. Une méthode selon l'une quelconque des revendications 1 à 10, dans laquelle le fractionnement par taille du mélange de fragments est effectué dans l'étape iv) par électrophorèse sur un gel dénaturant.

12. Une méthode selon la revendication 11, dans laquelle on visualise les fragments par un marqueur incorporé au cours de la synthèse du brin synthétique.

13. Une méthode selon la revendication 11, dans laquelle on visualise les fragments par hybridation avec une sonde marquée.

EP 0 227 772 B1

Fig.1

EMBODIMENT A

EP 0 227 772 B1

FIG.2
EMBODIMENT
B

A-TRACK     T-TRACK     C-TRACK     G-TRACK

2

INSERT

a)

FIG.3
EMBODIMENT
C

b)

∝SdATP

∝STTP

∝SdCTP

∝SdGTP

c)

c)

d)

d)

A-TRACK

T-TRACK

C-TRACK

G-TRACK

EP 0 227 772 B1

FIG.4.
EMBODIMENT
D

INSERT

a)

b)

∝SdATP    ∝STTP    ∝SdCTP    ∝SdGTP

c)

d)

e)

A-TRACK    T-TRACK    C-TRACK    G-TRACK

4

EP 0 227 772 B1

FIG.5
EMBODIMENT
E

A-TRACK        T-TRACK        C-TRACK        G-TRACK

5

EP 0 227 772 B1

FIG.6
EMBODIMENT F

A-TRACK    T-TRACK    C-TRACK    G-TRACK

EP 0 227 772 B1

FIG.7

EMBODIMENT
G

a)

b)

$3'$ $5'$

c)

$S$  $S$  $5'$

d)

$S$  $S$  $5'$

e)

N-TRACK

FIG.8 EMBODIMENT
H

g)
N-TRACK